# EUROPEAN PATENT SPECIFICATION

(11) **EP 2 978 732 B1**
(45) Date of publication and mention of the grant of the patent: **01.02.2017**
(21) Application number: 13711913.7
(22) Date of filing: 26.03.2013
(51) Int. Cl.: C07C 1/12, C10L 3/08, C25B 15/08, C25B 1/04

(54) **METHOD AND PLANT FOR THE PRODUCTION OF METHANE**
VERFAHREN UND ANLAGE ZUR HERSTELLUNG VON METHAN
PROCÉDÉ ET INSTALLATION POUR LA PRODUCTION DE MÉTHANE

(43) Date of publication of application: 03.02.2016
(73) Proprietor: Outotec (Finland) Oy, 02230 Espoo (FI)
(72) Inventor: ANASTASIJEVIC, Nikola, 63674 Altenstadt (DE); VON GARNIER, Agnes, 61440 Oberursel (DE); ORTH, Andreas, 61381 Friedrichsdorf (DE); SCHAAF, Tanja, 60439 Frankfurt am Main (DE); SCHUSTER, Markus, 70329 Stuttgart (DE); STRÖDER, Michael, 60438 Frankfurt am Main (DE)
(74) Representative: Keil & Schaafhausen Patent- und Rechtsanwälte PartGmbB
(86) International application number: PCT/EP2013/056384
(87) International publication number: WO 2014/154250

(56) References cited:
- US-A- 3 766 027
- US-A- 5 128 003

## Description

The present invention relates to a method and a plant for the production of methane, wherein hydrogen is produced from water in an electrolysis device and in a succeeding heterogeneously catalyzed reaction the hydrogen together with carbon dioxide is converted to methane.

In times of increasing shortage of fossil raw materials and the urgent necessity of a reduction of the CO₂ emission (carbon capture) to reduce global warming, a change to renewable energies is inevitable. However, if the energy supply is effected by renewable energies to an increasing extent, excesses from renewable energies, such as by solar and wind power stations, must be stored, so that peak loads in the energy demand can be compensated.

One such possibility for energy storage is the so-called renewable methane concept. This is based on the bidirectional coupling of electricity and natural gas network. Regenerative current electrolytically breaks down water into is constituents hydrogen and oxygen. With CO₂, which can be obtained from the atmosphere, industrial processes, still existing fossil power plants or biomass, the hydrogen then is converted to methane in a thermochemical synthesis, also referred to as methanation (Sabatier process). The renewable methane subsequently is stored, transported and depending on requirement used e.g. as fuel in gas or gas and steam power plants as balancing and reserve energy via a reconversion to electricity. In this way, decentrally generated regenerative current is converted into a CO₂-neutral energy carrier with high energy density.

If the reconversion of renewable methane is effected decentrally in heat-controlled block-type thermal power plants, the degree of utilization by combined heat and power generation can further be increased and households can be supplied with an emission-neutral natural gas substitute (principle of decentralized power generation: Many decentral block-type thermal power plants utilize "green methane" and are controlled centrally).

In addition, renewable methane can be used in the heat and traffic sector, i.e. for example for providing high-temperature process heat, in passenger cars or in hybrid vehicles as so-called "range extender" to increase the range. Beside methane, further fuels such as dimethyl ether or renewable kerosine can also be produced from hydrogen and carbon dioxide, which can be used in long-distance traffic (airborne, waterborne and goods traffic).

The decisive advantage as compared to pure hydrogen concepts is the utilization of the existing infrastructure, such as gas networks, gas accumulators and end-user devices. Technologies for natural gas are state of the art and conventionally available, which for the hydrogen technology is the case only to a limited extent. Furthermore, methane has a three times higher energy density and thus consumes three times less space than hydrogen. Finally, the risk of explosion of methane also is lower than that of hydrogen. Anyway, hydrogen production is the precondition for the methanation process.

As mentioned already, the electrochemical production of hydrogen is carried out by electrolysis. The principle of electrolysis is based on the fact that through two electrodes a direct electrical current is introduced into a conductive liquid, also referred to as electrolyte. Due to the electrolysis, reaction products are obtained at the electrodes from the substances contained in the electrolyte:
The voltage source effects an electron deficiency at the electrode connected with the positive pole (anode) and an electron excess in the other electrode connected with the negative pole (cathode). The two electrodes are separated by a diaphragm or a membrane. The liquid between the cathode and the anode is called electrolyte.

Within the water electrolysis process, water is split into oxygen and hydrogen according to the following reaction:

2H₂O → 2 H₂ + O₂.

At the cathode, water is decomposed into hydrogen and hydroxide-ions by consumption of electrons:

2 H₂O + 2e⁻ → H₂ + 2 OH⁻.

The hydroxide ions form water and oxygen at the anode under release of electrons:

4 OH⁻ → 2 H₂O + O₂ + 4 e⁻.

To prevent an intermixture of the resulting gases hydrogen and oxygen, the anode and the cathode space are separated from each other by a porous diaphragm or membrane. However, oxygen impurities occur in the hydrogen obtained, which are formed because the used separators between anode and cathode of the electrolysis are not completely gas-tight. The oxygen content of the hydrogen gas should be adjusted between 0 and 2 vol-% depending on the process condition, which corresponds to 50 % of the lower explosion limit. Values above 2 vol-% therefore are not admissible for this system and in continuously operating electrolysis devices they are avoided by an automated safety shut-down.

Even if the contained oxygen thus does not involve a risk of explosion, the residual amount of oxygen nevertheless reduces the gas purity and can deactivate catalysts in downstream processes by oxidation. It is therefore common practice that before a further use of the hydrogen the oxygen is removed in that it is catalytically converted to water with the hydrogen.

A further impurity of the hydrogen produced by electrolysis of water is moisture. The same is present in the resulting total product stream, because the hydrogen is saturated with steam, with the degree of saturation being greatly temperature-dependent. Usually, the hydrogen therefore is liberated from water in drying processes, wherein here adsorption dryers generally are used, which are filled with a drying agent such as silica gel, and which adsorb the contained water and thus remove it from the hydrogen. Two drying units are connected parallel to each other, so that the hydrogen stream always can be passed over one of the two units, while the respectively other unit is regenerated, whereby despite drying in a batch process a continuous procedure can be ensured.

The previously known methanation process also requires dry hydrogen without further impurities, so that the catalyst used for the methanation is protected against deactivation, in particular against oxidation by contained oxygen. In the article "Erneuerbares Methan. Eine Lösung zur Integration und Speicherung Erneuerbarer Energien und ein Weg zur regenerativen Vollversorgung" by Michael Sterner, Michael Specht et al, published in IRES, 2009, pages 51ff a typical methanation plant is presented. In this plant, methods for the removal of oxygen and water are connected in series, in order to ensure the required methane purity.

However, the production of renewable methane (including H₂ production) requires an expensive equipment and is difficult to regulate or control, so that the method becomes less economic.

Document US 5,128,003 A upon which the preamble of claim 1 is based discloses a method for producing oxygen and methane from carbon dioxide and hydrogen utilizing a methanation reactor, a reforming reactor and an electrolyzer.

Document US 3,766,027 A discloses a process of fixation and conversion of carbon dioxide from the atmosphere of other sources to produce methane and oxygen. Carbon dioxide is corrupt from a CO₂-containing source and separated by chemical concentration. A special cell is provided in which hydrogen is produced and reacted with the separated CO₂ at methanation conditions to produce methane.

It is the object of the present invention to provide a method in which a coupling between electrolysis of water and methanation is effected with a simplified purification of the hydrogen resulting from the electrolysis and a simple process management.

This object is solved by a method with the features of claim 1. In this method hydrogen is generated from water by electrolysis. From the electrolysis process a total product stream is withdrawn, which beside hydrogen also contains oxygen in an amount between 0.001 and 2 vol-% and water (saturated gas). This total product stream is supplied to a reactor, in which is at least one methanation stage the hydrogen is converted to methane with carbon dioxide in a heteroge-neously catalyzed reaction.

For producing the hydrogen gas from water, preferably an alkaline electrolyte, quite particularly preferably an aqueous potassium hydroxide solution is used as electrolyte. It is, however, also possible to use acid electrolytes, such as H₂SO₃, which is obtained in the production of SO₂.

The invention is based on the finding that the contained steam leads to the fact that the strongly exothermal reaction is slowed down and this impurity hence is not disturbing, but rather can be utilized for moderating the methanation.

It has also been found that the removal of oxygen by catalytic oxidation of the oxygen with hydrogen to obtain water can take place inside the methanation reactor, which has the advantage that expensive intermediate apparatuses for purification of the gas stream can be omitted completely. In accordance with a development of the invention, a further catalytic stage therefore is provided inside the methanation reactor, in which the removal of oxygen from the hydrogen is effected by catalytic oxidation. Preferably, the CO₂ likewise required as educt for the methanation process is fed in downstream of this stage to avoid side reactions at the catalyst. It is advantageous that the energy released by catalytic oxidation can be utilized to further heat up the entire gas stream, so that the necessary activation energy for starting the methanation reaction can at least partly be provided inside the reactor, without this amount of energy having to be provided by an external source.

When the catalyst of the methanation stage is chosen such that the contained oxygen impurities of up to 2 vol-% do not damage the same within a short downtime, the methanation stage itself also can be utilized as stage for oxygen removal, which means that here the catalyst for oxygen removal and the catalyst for methanation are arranged in a common stage.

As catalyst for oxygen removal, palladium preferably is used, since the same completely converts the oxygen during long downtimes.

Preferably, the reaction for oxygen removal is carried out at a temperature between 300 and 350 °C, in particular if the oxygen removal is effected in a stage separate from the methanation. At this temperature, a damage of the catalyst reliably is avoided and at the same time a complete conversion is ensured.

The reaction temperature in the methanation stage is between 250 and 300 °C for low temperature methanation and between 450 and 500 °C for high temperature methanation.

As heterogeneous catalyst for the methanation stage nickel preferably is used, since this catalyst is suitable for high conversions of the hydrogen with a high yield of methane at the same time. In addition, nickel is comparatively insensitive to gaseous impurities, which are introduced by the feed of the methanation, so that here an oxygen removal is not absolutely necessary.

Furthermore, it was found to be advantageous to admix steam to the feed of at least one of the methanation stages. This steam acts as moderator of the strongly exothermal methanation reaction. A "runaway" of the reaction thereby can be avoided, in which due to a local increase in temperature so-called hot spots, i.e. locally very hot points are formed, at which due to the larger amount of energy provided strongly exothermal reactions preferably take place, which in turn leads to a further temperature increase of the system and to undesired conversions, until the reaction can no longer be regulated or controlled. At the end of such process the explosion of the reactor occurs. It is therefore desirable to throttle the reaction velocity and rate within one stage, in order to ensure a safe operation. It is of course particularly advantageous here when due to the electrolysis and/or the conversion of the oxygen, amounts of water already are contained in the hydrogen and thus only small amounts of water must be supplied from outside. The supplied quantity can be effected for example by continuous measurements of the hydrogen content and matching with a firmly set control value.

Furthermore, it was found to be favourable to again admix parts of the already converted gas to the feed of this methanation stage after passing through at least one methanation stage. The gas which is passed over the methanation stage thereby is diluted, whereby reaction velocity and rate likewise are reduced.

Both the throttling of the reaction by supplying steam and the throttling by recirculating a partial stream of the product stream exiting from the methanation are used in particular for controlling the first methanation stage, as here the risk of a runaway of the reaction due to the highest concentration of the educts hydrogen and carbon dioxide is particularly high.

Of course, the reaction also can be regulated by using both methods in combination, i.e. supplying steam and recirculating a partial stream of the product stream.

If a part of the product stream exiting from a methanation stage is recirculated, it was found to be particularly favourable that a compressor brings this recirculated stream to that pressure of about 20 bar with which the total product stream from the electrolysis is fed into the methanation reactor, whereby pressure losses (1%, that is about 200 mbar) inside the reactor are compensated.

Finally, it is the subject-matter of the invention that the removal of water also is effected inside the methanation reactor. Due to the spatial arrangement, the use of the usual adsorption methods however is not recommendable here, but instead a continuous condensation. The hydrogen is compressed to up to 2.5 MPa and at the same time cooled down to not more than 10 °C. Due to this increased pressure and the decreased temperature, contained water will condensate out. The moisture remaining in the hydrogen then amounts to less than 0.1 vol-%. A required gas purity of 99.91 % can be achieved by using a pressure of 2.0 MPa and a gas temperature of 15 °C.

To further raise the quality of the methane obtained, it is of course possible to subsequently provide a removal of oxygen and/or water in the methanation. In particular the removal of water may be necessary when steam has been admixed for throttling the reaction.

For removing the water, both the known adsorption method can be employed after the methanation and the methane can be dried by a continuous condensation.

The invention furthermore comprises a plant with the features of claim 10, which is suitable for carrying out the method according to the invention. Such plant comprises an electrolysis device in which hydrogen is generated from water by electrolysis, and a reactor in which the hydrogen is converted to methane in at least one methanation stage in a heterogeneously catalyzed reaction with carbon dioxide. The reactor and the electrolysis device are connected with each other via a conduit through which a total product stream, which beside hydrogen also contains oxygen, water and carbon dioxide, is guided from the electrolysis device into the reactor. This conduit can be guided through a heat exchanger, whereby the total product stream is brought to the required reaction temperature for the reactions taking place inside the reactor.

Besides the methanation stage the reactor also contains at least one stage in which oxygen is converted in a heterogeneously catalyzed reaction. In a particularly preferred embodiment, the methanation stage and the stage for removing the oxygen coincide locally, in that both the catalyst for methanation and the catalyst for oxygen removal are arranged one beside the other or mixed. As a result, the plant can be built even more compact, but this necessitates that the catalyst for methanation is not damaged by the contained oxygen impurities of up to 2 vol-%. This is the case for example when using nickel.

A further preferred embodiment provides that the at least one stage for removing the oxygen is arranged above the at least one methanation stage and the conduit for supplying the total product stream opens into the reactor above the at least one stage for oxygen removal. Due to this arrangement, the gas stream is heated further inside the reactor before entry into the first methanation stage due to the reaction of the oxygen.

A preferred aspect of the invention provides that at least one further reactor with at least one further methanation stage is provided downstream of the reactor. This has the advantage that the reaction need not take place completely inside the first reactor, but inside each reactor only partial conversions are operated. This reduces the risk of the runaway of the reaction, in particular since between the individual reactors heat exchangers can easily be provided, whereby heat is withdrawn from the system.

Finally, it was found to be favourable when the reactor contains the heterogeneous catalyst for the methanation and/or for the oxygen removal as fixed bed or as coating of the walls. As a result, the catalyst can easily be incorporated and nevertheless is uniformly swept over by the gas stream, whereby a rather complete conversion can be ensured.

Further features, advantages and possible applications of the invention can also be taken from the following description and the drawings. All features described and/or illustrated form the subject-matter of the invention per se or in any combination, independent of their inclusion in the claims or their back-references.

In the drawings:
- Fig. 1: shows a schematic representation of the energy cycle for the storage of energy by methanation;
- Fig. 2: shows a schematic representation of a method for methanation according to the prior art;
- Fig. 3: shows a schematic representation of the method according to the invention; and
- Fig. 4: shows a further schematic representation of the method according to the invention.

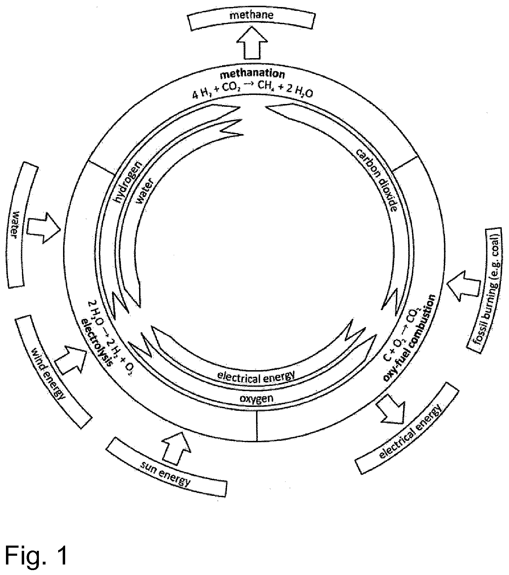

Fig. 1 shows the energy cycle through which renewable energy can be stored by methane formation. Water is introduced into the electrolysis together with wind and/or solar energy, whereby hydrogen and oxygen are obtained. The oxygen can be used to convert fossil fuels, such as e.g. coal, to CO₂. During this conversion, electric energy is released.

The hydrogen likewise obtained in the electrolysis together with carbon dioxide and in part also with water is converted to methane in the methanation according to the reaction equations indicated in Fig. 1. The entire process hence proceeds in a carbon-dioxide-neutral manner, provides electric energy during the combustion and stores energy in the methane produced.

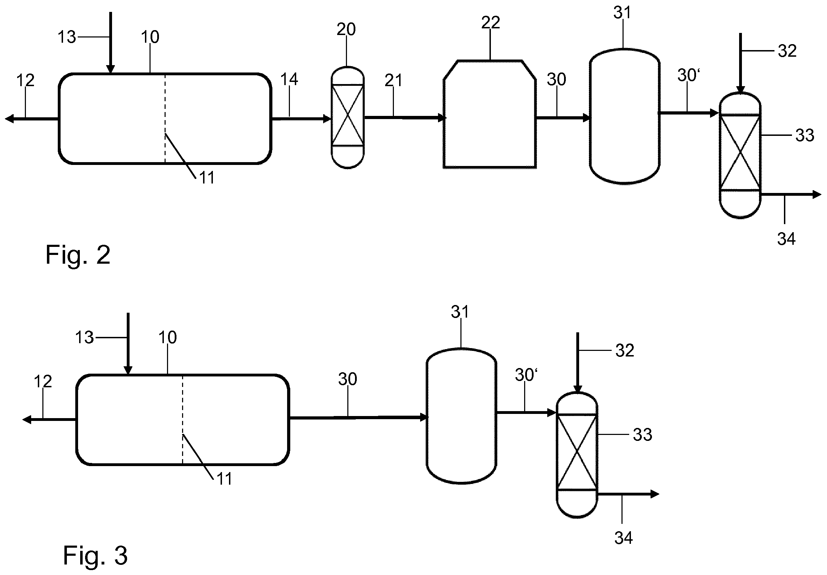

Fig. 2 shows a method for producing methane from hydrogen, which is obtained by an electrolysis, as it is known from the prior art. In an electrolysis device 10 with a diaphragm 11 water is electrolytically converted to hydrogen and oxygen. The oxygen produced is withdrawn via conduit 12. Via conduit 13 it is possible to refill the electrolyte, preferably an aqueous solution of KOH. Via conduit 14, a total product stream of the electrolysis is withdrawn, which beside hydrogen also contains oxygen in an amount of up to 2 vol-%, water and carbon dioxide.

This total product stream is supplied to a reactor 20, in which the heterogeneously catalyzed oxygen is removed from the total product stream, in that it is converted to steam with hydrogen. The total product stream, which now is liberated from oxygen, is supplied to a drying device 22 via conduit 21. Said drying device preferably is an adsorber in batch operation, in which the contained moisture is removed from the gas by an adsorbent, for example silica gel. To be able to ensure a continuous method despite the batch operation, two adsorbers generally are connected in parallel, so that always one adsorber can be utilized for removing moisture, while the other adsorber is regenerated.

Via conduit 30, the dehumidified hydrogen finally is supplied to a hydrogen tank 31, before it is fed into the methanation reactor 33 via conduit 30'. Via conduit 32, carbon dioxide additionally is fed into this reactor 33. On a heterogeneous catalyst, hydrogen and carbon dioxide are converted to methane and water. The product mixture obtained is withdrawn via conduit 34.

Fig. 3 shows the fundamental principle of the present invention. Here as well, oxygen and hydrogen are produced in the electrolysis device 10 with the diaphragm 11. The oxygen is withdrawn via conduit 12, while an alkaline electrolyte for increasing the conversion can be introduced via conduit 13. Via conduit 30, a total product stream which beside hydrogen also contains oxygen, water and carbon dioxide is withdrawn and supplied to a hydrogen tank 31. From this tank, the total product stream subsequently is fed into the reactor 33 through conduit 30' and mixed there with the carbon dioxide supplied via conduit 32.

On at least one heterogeneously catalyzed stage inside the reactor, hydrogen and carbon dioxide are converted to methane and water and the resulting product mixture is withdrawn via conduit 34. Such method only can be carried out when the catalyst is insensitive to oxidation reactions with the still contained oxygen, which is the case for example when nickel is used as catalyst. In such reaction control it is advantageous that the water contained in the total product stream leads to the fact that the equilibrium of the reaction is shifted more towards the educt side and thus the risk of the runaway of the reaction is reduced or completely excluded.

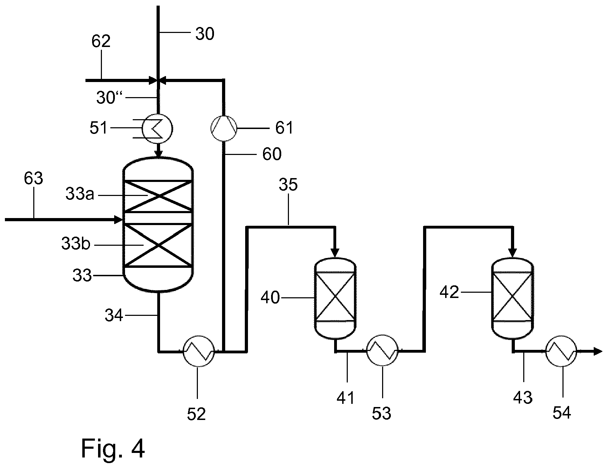

Fig. 4 finally shows a preferred embodiment of the present invention. Via conduit 30, the total product stream is introduced into the reactor 33, wherein the same has previously been heated by the heat exchanger 51 preferably to a temperature between 300 and 350 °C. In the reactor 33, at least one heterogeneous catalyst stage 33a is present, in which the oxygen reacts with hydrogen to form water. The catalyst stage 33a is arranged inside the reactor 33 such that the total product stream first passes this catalyst stage 33a. Downstream of the catalyst stage 33a for removing the oxygen at least one catalyst stage 33b is provided for methanation of the mixture of hydrogen and carbon dioxide. CO₂ for methanation is admixed between the first catalyst stage 33a and the second catalyst stage 33b via conduit 63.

Via conduit 34, the product mixture obtained in the methanation is withdrawn and cooled further in the heat exchanger 52. Via conduit 35, the product mixture then can partly or completely be supplied to a further, separate reactor 40, in which there is also provided at least one further methanation stage. The total conversion can further be increased thereby.

Via conduit 41, the partly methanized product stream is supplied to the heat exchanger 53, in which the product mixture is cooled again, before it is introduced into a reactor 42 in which there is also provided at least one methanation stage. By this circuitry very high total conversions can be achieved; by means of the interposed cooling steps, however, it can at the same time be prevented that the reaction runs away. The product stream from the last reactor 42 is withdrawn via conduit 43 and cooled in the heat exchanger 54.

To avoid the risk of the runaway of the reaction, it may furthermore be expedient to admix water to the total product stream 30 via conduit 62. By this active admixing of water, the equilibrium of the reaction can be shifted towards the educts and the reaction can thus be controlled. Due to the enrichment with water it may also be expedient to additionally provide a non-actuated condensation device between the individual reactors for methanation 33, 40 and 42 and thus again remove a dose of water from the respective product mixture before the succeeding stages for methanation, in order to ensure a complete conversion. Furthermore, it was found to be favourable to branch off a partial stream from the product stream exiting from the reactor 33 via conduit 60 and admix the same to the feed of the reactor 33, which is supplied via conduit 30. The supplied feed stream thereby is diluted, so that the locally obtained heat quantities can be limited. To compensate pressure losses via the reactor 33, which occur in particular with a plurality of catalyst stages, it is expedient to again compress the recirculated partial stream from conduit 60 to the reaction pressure via conduit 61.

### List of Reference Numerals

- 10: electrolysis device
- 11: diaphragm
- 12-14: conduit
- 20: reactor for oxygen removal
- 21: conduit
- 22: drying device
- 30: conduit
- 31: hydrogen storage tank
- 30': conduit
- 32: conduit
- 33: reactor for methanation
- 33a: stage for removal of oxygen
- 33b: stage for methanation
- 34, 35: conduit
- 40: reactor for methanation
- 41: conduit
- 42: reactor for methanation
- 43: conduit
- 51-54: heat exchanger
- 60: conduit
- 61: compressor
- 62, 63: conduit

## Claims

1. A method for producing methane, wherein hydrogen is generated from water by electrolysis, a total product stream is withdrawn from the electrolysis, which contains hydrogen, oxygen and water, and the total product stream is supplied to a reactor in which the hydrogen is converted to methane with carbon dioxide in at least one methanation stage in a heterogeneously catalyzed reaction, **characterized in that** beside at least one methanation stage the reactor also includes at least one stage in which the oxygen is removed in a heterogeneously catalyzed reaction, and that the total product stream first is guided over this at least one stage for oxygen removal and then over the at least one methanation stage, or that the stage for oxygen removal is the methanation stage itself.

2. The method according to claim 1, **characterized in that** as catalyst in the stage for oxygen removal palladium is used.

3. The method according to claim 1 or 2, **characterized in that** the reaction in the at least one stage for oxygen removal is carried out at temperatures between 300 and 350°C.

4. The method according to any of the preceding claims, **characterized in that** as catalyst in the methanation stage nickel is used.

5. The method according to any of the preceding claims, **characterized in that** before the at least one methanation stage steam is admixed to the total product stream.

6. The method according to any of the preceding claims, **characterized in that** parts of the stream exiting from a methanation stage are recirculated and admixed to the total product stream before the at least one methanation stage.

7. The method according to claim 6, **characterized in that** the recirculated partial stream is compressed to the reaction pressure.

8. The method according to any of the preceding claims, **characterized in that** inside the reactor for methane conversion water is removed by condensation.

9. The method according to any of the preceding claims, **characterized in that** a removal of oxygen and/or water is effected after the conversion to methane.

10. A plant for producing methane with an electrolysis device (10) in which hydrogen is generated from water by electrolysis, a reactor (33) in which the hydrogen is converted to methane in a heterogeneously catalyzed reaction in at least one methanation stage (33b), and a conduit (30) via which a total product stream, which contains hydrogen, oxygen and water, is guided from the electrolysis device into the reactor (33), **characterized in that** inside the reactor (33) beside the at least one methanation stage (33b) there is also provided at least one stage (33a) in which the oxygen is removed in a heterogeneously catalyzed reaction.

11. The plant according to claim 10, **characterized in that** the at least one stage (33a) for removing the oxygen is arranged above the at least one methanation stage (33b) and the conduit (30) opens into the reactor (33) above the at least one stage (33a) for oxygen removal.

12. The plant according to any of claims 10 to 11, **characterized in that** at least one further reactor (40, 42) with at least one further methanation stage is provided downstream of the reactor (33).

13. The plant according to any of claims 10 to 12, **characterized in that** the reactor (33) contains the heterogeneous catalyst of the methanation stage (33b) and/or of the stage (33a) for oxygen removal as fixed bed or as coating of the walls.

## Patentansprüche

1. Ein Verfahren zur Herstellung von Methan, wobei Wasserstoff durch Elektrolyse aus Wasser erzeugt wird, aus der Elektrolyse ein Gesamtproduktstrom abgezogen wird, der Wasserstoff, Sauerstoff und Wasser enthält, und wobei der Gesamtproduktstrom einem Reaktor zugeführt wird, in welchem der Wasserstoff mit Kohlendioxid in wenigstens einer Methanisierungsstufe in einem heterogen katalysierten Reaktor zu Methan umgesetzt wird, **dadurch gekennzeichnet, dass** neben der wenigstens einen Methanisierungsstufe der Reaktor außerdem wenigstens eine Stufe aufweist, in welcher der Sauerstoff in einer heterogen katalysierten Reaktion entfernt wird, und dass der Gesamtproduktstrom zunächst über diese wenigstens eine Stufe zur Sauerstoffentfernung und dann über die wenigstens eine Methanisierungsstufe geführt wird, oder dass die Stufe zur Sauerstoffentfernung die Methanisierungsstufe selbst ist.

2. Das Verfahren nach Anspruch 1, **dadurch gekennzeichnet, dass** als Katalysator in der Stufe zur Sauerstoffentfernung Palladium verwendet wird.

3. Das Verfahren nach Anspruch 1 oder 2, **dadurch gekennzeichnet, dass** die Reaktion in der wenigstens einen Stufe zur Sauerstoffentfernung bei Temperaturen zwischen 300 und 350°C durchgeführt wird.

4. Das Verfahren nach einem der vorhergehenden Ansprüche, **dadurch gekennzeichnet, dass** als Katalysator in der Methanisierungsstufe Nickel verwendet wird.

5. Das Verfahren nach einem der vorhergehenden Ansprüche, **dadurch gekennzeichnet, dass** vor der wenigstens einen Methanisierungsstufe dem Gesamtproduktstrom Dampf zugemischt wird.

6. Das Verfahren nach einem der vorhergehenden Ansprüche, **dadurch gekennzeichnet, dass** Teile des aus einer Methanisierungsstufe austretenden Stromes rezirkuliert und dem Gesamtproduktstrom vor der wenigstens einen Methanisierungsstufe zugemischt werden.

7. Das Verfahren nach Anspruch 6, **dadurch gekennzeichnet, dass** der rezirkulierte Teilstrom auf den Reaktionsdruck komprimiert wird.

8. Das Verfahren nach einem der vorhergehenden Ansprüche, **dadurch gekennzeichnet, dass** in den Reaktor zur Methanumwandlung Wasser durch Kondensation entfernt wird.

9. Das Verfahren nach einem der vorhergehenden Ansprüche, **dadurch gekennzeichnet, dass** ein Entfernen von Sauerstoff und/oder Wasser nach der Umwandlung in Methan durchgeführt wird.

10. Eine Anlage zur Herstellung von Methan mit einer Elektrolysevorrichtung (10), in welcher Wasserstoff durch Hydrolyse aus Wasser erzeugt wird, einem Reaktor (33), in welchem der Wasserstoff in einer heterogen katalysierten Reaktion in wenigstens einer Methanisierungsstufe (33b) zu Methan umgesetzt wird, und einer Leitung (30) über welche ein Gesamtproduktstrom, der Wasserstoff, Sauerstoff und Wasser enthält, von der Elektrolysevorrichtung in den Reaktor geführt wird, **dadurch gekennzeichnet, dass** in dem Reaktor (33) neben der wenigstens einen Methanisierungsstufe (33b) auch wenigstens eine Stufe (33a) vorgesehen ist, in welcher der Sauerstoff in einer heterogen katalysierten Reaktion entfernt wird.

11. Die Anlage nach Anspruch 10, **dadurch gekennzeichnet, dass** die wenigstens eine Stufe (33a) zum Entfernen des Sauerstoffs oberhalb der wenigstens einen Methanisierungsstufe (33b) angeordnet ist, und dass sich die Leitung (30) oberhalb der wenigstens einen Stufe (33a) zur Sauerstoffentfernung in den Reaktor (33) öffnet.

12. Die Anlage nach einem der Ansprüche 10 bis 11, **dadurch gekennzeichnet, dass** wenigstens ein weiterer Reaktor (40, 42) mit wenigstens einer weiteren Methanisierungsstufe stromabwärts des Reaktor (33) vorgesehen ist.

13. Die Anlage nach einem der Ansprüche 10 bis 12, **dadurch gekennzeichnet, dass** der Reaktor (33) den heterogenen Katalysator der Methanisierungsstufe (33b) und/oder der Stufe (33a) zur Sauerstoffentfernung als Festbett oder als Beschichtung der Wände aufweist.

## Revendications

1. Procédé pour la production de méthane, dans lequel de l'hydrogène est généré à partir d'eau par électrolyse, un courant de produit total est soutiré à partir de l'électrolyse, qui contient de l'hydrogène, de l'oxygène et de l'eau, et le courant de produit total est introduit dans un réacteur dans lequel l'hydrogène est converti en méthane avec du dioxyde de carbone dans au moins une étape de méthanisation au cours d'une réaction catalysée de manière hétérogène, **caractérisé en ce qu'**au moins à part une étape de méthanisation, le réacteur comprend également au moins une étape dans laquelle de l'oxygène est éliminé au cours d'une réaction catalysée de manière hétérogène, et que l'écoulement de produit total est premièrement guidé au cours de cette au moins une étape d'élimination d'oxygène et ensuite au cours de l'au moins une étape de méthanisation, ou que l'étape d'élimination d'oxygène est l'étape de méthanisation elle-même.

2. Procédé selon la revendication 1, **caractérisé en ce que** du palladium est le catalyseur utilisé dans l'étape d'élimination d'oxygène.

3. Procédé selon la revendication 1 ou la revendication 2, **caractérisé en ce que** la réaction au cours de l'au moins une étape d'élimination d'oxygène est effectuée à des températures comprises entre 300 et 350 °C.

4. Procédé selon l'une quelconque des revendications précédentes, **caractérisé en ce que** du nickel est le catalyseur utilisé dans l'étape de méthanisation.

5. Procédé selon l'une quelconque des revendications précédentes, **caractérisé en ce qu'**avant l'au moins une étape de méthanisation un courant est mélangé au courant de produit total.

6. Procédé selon l'une quelconque des revendications précédentes, **caractérisé en ce que** les parties du courant sortant d'une étape de méthanisation sont remises en circulation et mélangées au courant de produit total avant l'au moins une étape de méthanisation.

7. Procédé selon la revendication 6, **caractérisé en ce que** le courant partiel remis en circulation est comprimé à la pression de réaction.

8. Procédé selon l'une quelconque des revendications précédentes, **caractérisé en ce qu'**à l'intérieur du réacteur destiné à la conversion de méthane, de l'eau est éliminée par condensation.

9. Procédé selon l'une quelconque des revendications précédentes, **caractérisé en ce que** l'élimination d'oxygène et/ou d'eau est effectuée après la conversion en méthane.

10. Installation pour la production de méthane comprenant un dispositif d'électrolyse (10) dans lequel de l'hydrogène est généré à partir d'eau par électrolyse, un réacteur (33) dans lequel l'hydrogène est converti en méthane dans une réaction catalysée de manière hétérogène au cours d'au moins une étape de méthanisation (33b), et une canalisation (30) par laquelle un courant de produit total, qui contient de l'hydrogène, de l'oxygène et de l'eau, est guidé depuis le dispositif d'électrolyse dans le réacteur (33), **caractérisé en ce qu'**à l'intérieur du réacteur (33) à part l'au moins une étape de méthanisation (33b), on fournit également au moins une étape (33a) dans laquelle l'oxygène est éliminé dans une réaction catalysée de manière hétérogène.

11. Installation selon la revendication 10, **caractérisée en ce que** l'au moins une étape (33a) d'élimination d'oxygène est agencée au-dessus de l'au moins une étape de méthanisation (33b) et la canalisation (30) s'ouvre dans le réacteur (33) au-dessus de l'au moins une étape (33a) d'élimination d'oxygène.

12. Installation selon l'une quelconque des revendications 10 et 11, **caractérisée en ce qu'**au moins un réacteur (40, 42) supplémentaire avec au moins une étape de méthanisation supplémentaire est introduit en aval du réacteur (33).

13. Installation selon l'une quelconque des revendications 10 à 12, **caractérisée en ce que** le réacteur (33) contient le catalyseur hétérogène de l'étape de méthanisation (33b) et/ou de l'étape (33a) d'élimination d'oxygène en tant que lit fixe ou en tant que revêtement des parois.
